# EUROPEAN PATENT APPLICATION

(11) **EP 1 967 197 A1**
(43) Date of publication of application: **10.09.2008**
(21) Application number: 07004869.9
(22) Date of filing: 09.03.2007
(51) Int. Cl.: A61K 36/00

(54) **Use of preparations, purifications and extracts of aloe**

(71) Applicant: Cognis IP Management GmbH, 40589 Düsseldorf (DE)
(72) Inventor: Buchwald-Werner, Sybille, Dr., 40589 Düsseldorf (DE)
(74) Representative: Fabry, Bernd

(57) **Abstract**

Suggested is the use of preparations, purifications and/or extracts of Aloe as dietary supplements or functional food.

## Description

### Field of the invention

The present invention is related to the area of botanical extracts; more particularly it refers to the use of certain plant preparations or extracts as dietary supplements and/or functional food.

### Background of the invention

Food is essential to life. It adds to the richness of the human experience. Yet, people tend to eat too much, and limiting consumption can affect the quality and length of the life. There are known factors that help extend life, and there are at least as many that can shorten it, one of the negative factors being over consumption of foods. It is common sense that while under nutrition remains a problem in many parts of the world, the more likely problem for Europeans and Americans is the tendency to eat too much. The type and amount of foods are implicated in weight control and general health.

People know that they should try to eat less, but many have difficulty given the typical western lifestyle in which people can eat as much as they want. Healthy, sustainable weight management isn't just about how many calories you eat; it's also about eating food that's great tasting and satiating, so a person doesn't tend to overeat. Over a recent passage of twenty years, obesity increased to epidemic levels. According to the National Institute of Diabetes and Digestive and Kidney Diseases (NIDDK), about 64% of adults (130 million adults over the age of 20) are overweight and 30% (61 million adults) are obese. Amongst children, the rate of increase in obesity is especially alarming. Between 1980 and 2000, the percentage of overweight children more than doubled to 15%.

Millions of Europeans and Americans are dieting and will continue to invest their money to lose weight. Unfortunately, much of their investment produces little gain toward a lower weight and better health. Weight control and diet formulations have been one area where weight conscious individuals have looked, however, these formulations are often not perceived as healthy. In fact, certain well-known products, like for example those containing ephedrine alkaloids, citing a link to increased risk in heart attacks and strokes. This action left many weight conscious consumers without their favourite diet medication and further started a controversy surrounding dietary supplements. Many are justifiably wary of unfamiliar, new dietary supplements and functional foods that promise results that seem unreasonably good.

The cyclic emergence of fad diets has preyed upon the millions of people who are trying to lose weight or prevent weight gain. What is needed, however, is an approach that provides a reliable moderator to the person wanting to truly enjoy food as one of the joys of life. Diet supplements, pills or regimens that are intended as stand-alone solutions to weight loss and weight management are not available with a simple self regulatory feature. In all cases weight control consistent with health should be part of an integrated weight loss and weight management plan which includes healthy eating and regular exercise. What is needed is a food supplement, which, instead of making unrealistic and false weight loss claims, will help a weight conscious individual with appetite control.

Understanding food consumption in terms of digestion and satiety is not a matter simply summarized or outlined. The development of effective diet foods and regimens has lagged behind breakthrough understandings based upon a vast amount of research. There is a burning desire for the achievement of some means for people to be able to enjoy food for what it adds to life while sustaining it, while not overeating on a regular basis. For example, it is well known that the gastrointestinal presence of ingested nutrients initiates a range of physiological responses that serve to facilitate the overall digestive process. During a meal, ingested nutrients accumulate in the stomach, with a significant portion passing on to the small intestine. Thus, peptides and transmitters are released, and various neural elements are activated that coordinate gastrointestinal secretion and motility and can eventually lead to meal termination or satiety. Consumption of food in general will cause a person to feel full or satiated as eating progresses. Once a person is satiated, the inclination moves from eating to rest from eating. Proteins, minerals, fibre, carbohydrates, and fats all have effects on this phenomenon. Satiety can be measured both subjectively by noting what a subject feels like and analytically by looking at certain biochemical markers.

Likewise, fibre is a satiating food, if not macronutrient. Cholecystokinin is associated with satiety. Fat stimulates cholecystokinin release, and fibre appears to prolong cholecystokinin elevation during the alimentary period. Studies show that in women, the feeling of satiety caused by cholecystokinin release is enhanced by increasing either the fibre or fat content of a low-fat, low-fibre meal. In the men, the increase in cholecystokinin concentration did not differ between meals, but the two low-fat meals elicited a greater feeling of satiety than did the high-fat meal. Also various herbal remedies have been suggested in micro amounts as providing a wealth of health benefits, as have some mineral supplements. For example, green tea polyphenols, especially the catechin, epigallocatechin gallate, or EGCG-caffeine mixture appears to have potential benefits in the treatment of obesity. In several studies an enhancement in dietary fat oxidation and dietary induced thermogenesis (DIT was shown), which could result in weight reduction. In addition Green tea catechins have been proposed as a cancer chemo-preventative based on a variety of laboratory studies. Note that that both effects are stimulated by different parts of the human body: while satiety represents a response from the stomach, reduction in appetite is linked to process taking place in the brain.

Therefore, the problem underlying the present invention has been to identify active agents, preferably from natural sources, which simultaneously support satiety and/or reduce appetite when taken up orally by humans so that they can act both as dietary supplement and functional food. It is superfluous to mention that these new active agents must not cause unwanted side effects.

### Detailed description of the invention

The present invention refers to the use of preparations, purifications and/or extracts of Aloe as dietary supplements or functional food.

Surprisingly it has been observed in several in-vitro experiments that preparation, purifications and extracts of Aloe support satiety and reduce appetite when taken up orally by humans. Since the products are toxicologically safe and do not cause side effects, with the exception of some well known other advantageous properties which provide additional benefits, the use of these preparations and extracts solves the problem underlying the present invention in a perfect manner.

### Aloe

Aloe, also written *Aloë,* is a genus containing about four hundred species of flowering succulent plants. The genus is native to Africa and is common in South Africa's Cape Province and the mountains of tropical Africa, and neighbouring areas such as Madagascar, the Arabian peninsula and the islands off Africa. The APG II system (2003) placed the genus in the family Asphodelaceae. In the past it has also been assigned to families Aloaceae and Liliaceae. Members of the closely allied genera *Gasteria, Haworthia* and *Kniphofia* which have a similar mode of growth, are also popularly known as Aloes.

*Aloe* species are frequently cultivated as ornamental plants both in gardens and in pots. Many *Aloe* species are highly decorative and are valued by collectors of succulents. Some species, in particular *Aloe vera* are purported to have health supporting properties. Other uses of Aloes include their role in alternative medicines and in home first aid. Both the translucent inner pulp as well as the resinous yellow juice of the Aloe plant is used *externally* to relieve skin discomforts and *internally* as a laxative. To date, some research has shown that Aloe vera produces positive active benefits for healing damaged skin. Conversely, other research suggests Aloe vera can negatively effect healing. Some Aloe species, preferably Aloe vera barbadensis have also been used for human consumption. For example, drinks made from or containing chunks of Aloe pulp are popular in Asia as commercial beverages and as a tea additive; this is notably true in Korea. Aloe contains a number of active substances used as a purgative. The active substance is produced from various species of Aloe, such as *A. vera, A. vulgaris, A. socotrina, A. chinensis,* and *A. perryi,* Aloe ferox. Aloes (so-called Aloin or Barbaloin) is the expressed juice of the leaves of the plant. When the leaves are cut, the juice that flows out is collected and evaporated. In addition the natural gel is used, which is the translucent inner pulp, obtained by separated the skin of the leave as well as the resinous yelloe juice right under the skin. The pulp is mechanically converted into a gel. There have been few properly conducted studies about possible benefits of Aloe gel taken internally. One study found improved wound healing in mice. Another found a positive effect of lowering risk factors in patients with heart disease. Some research has shown decreasing fasting blood sugar in diabetic animals given Aloe. Recently, several studies showed that oral administration of Aloe protects against nervous stomach syndrome and improves the condition of peptic ulcers.2 It has been demonstrated that Aloe vera gel may protect against and reduce intestinal inflammation. Moreover, Aloe vera gel is believed to moderate gastric acid secretion, overproduction of which may lead to reflux and stomach irritation.

Aloe has been marketed as a remedy for coughs, wounds, ulcers, gastritis, diabetes, cancer, headaches, arthritis, immune-system deficiencies, and many other conditions when taken internally. The preparations and extracts of Aloe can be obtained according to the procedures well known from the state of the art. They may be present in the final compositions in amounts of 0.1 to 99 % b.w., preferably 1 to 30 % b.w. and most preferably 2 to 10 % b.w. in the form of a gel or a powder, calculated to the natural gel content. For example, a 0.02 % b.w. concentration (200:1) would be indicated with 4 % b.w.

### Plant preparations, purification and extracts

In another preferred embodiment of the present invention the preparations, purifications or extracts of Aloe can be combined with extracts of other plants which are well known for their advantageous properties in food compositions. Typically, the plant extracts according to the present invention are chosen from the plants selected from the group consisting of *Ginkgo biloba, Oleacea europensis, Trifolium pratense, Litchi sinensis, Vitis vinifera, Brassica oleracea, Punica granatum, Petroselinium crispum, Passiflora incarnata, Medicago sativa, Valeriana officinalis, Castanea sativa, , Hapagophytum procumbens, Melissa officinalis, Panax ginseng, Camellia sinensis, Paullinia cupana, Opuntia ficus-indica, Caralluma Fimbriata* and *Hoodia gordonii.* Particularly useful are extracts of Rooibos (Aspalathus linearis L).and Honey bush, *(Cyclopia ssp L.)* In the following short summaries of the composition and the main constituents of the cited extracts is given.

### Ginkgo biloba

The active ingredients of extracts from the leaves of the ginkgo tree (*Ginkgo biloba*) are flavonoid glycosides, which among others contain (iso)quercitin glycosides, kaempferol, kaempferol-3-rhamnosides, isorhamnetin, luteoline glycosides, sitosterol glycosides and predominantly hexacyclic terpene lactones, consisting of ginkgolides A, B, C, J, M and bilobalides.

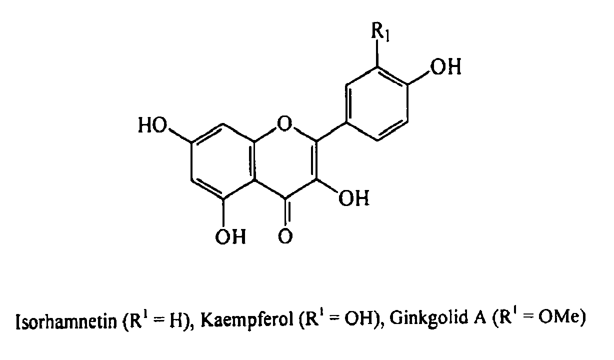

### Camellia sinensis

Leaves of green tea contain many compounds, such as polysaccharides, volatile oils, vitamins, minerals, purines, alkaloids (e.g. caffeine) and polyphenols (catechins and flavonoids). Although all three tea types have antibacterial and free radical capturing (antioxidising) activities, the efficacy decreases substantially the darker the variety of tea is. This is due to the lower contents of anti-oxidising polyphenols remaining in the leaves. Among the various components of green tea extracts, polyphenols of the flavonoid and catechin type ("tea tannins") are the most important.

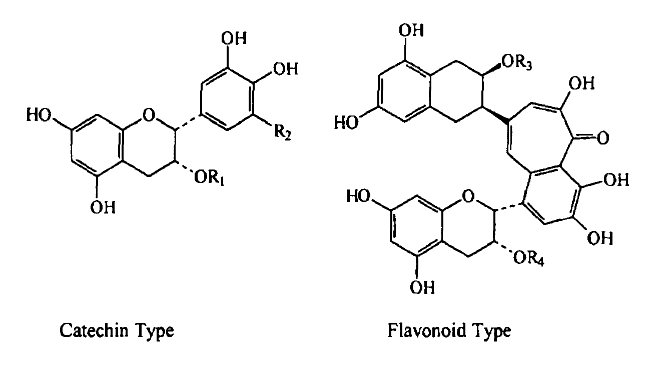

| **Components** | **R1** | **R2** | **R3** | **R4** |
|---|---|---|---|---|
| (-)-Epicatechin | H | H | | |
| (-) Epigallocatechin | H | OH | | |
| (-) Epicatechin gallate | Galloyl | H | | |
| (-) Epigallocatechin gallate | Galloyl | OH | | |
| Theflavin | | | H | H |
| Theaflavin monogallate A | | | Galloyl | H |
| Theaflavin monogallat B | | | H | Galloyl |
| Theaflavin digallate | | | Galloyl | Galloyl |

### Oleacea europensis

The main constituent of the leaves of the olive tree (*Oleacea europensis*) is the antioxidant oleuropein, which is also the main source for hydroxytyrosol.

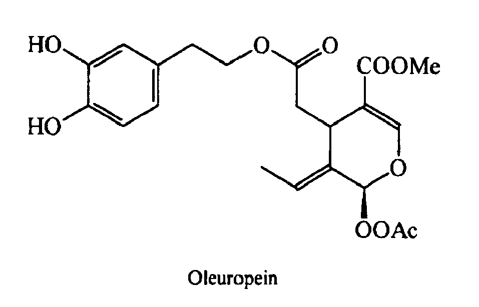

### Trifolium pratense

The main active principles of red clover (*Triflolium pratense*) are isoflavones, like e.g. daidzein, genestein, formononentin and biochanin as well as their glucosides like ononin or sissostrin:

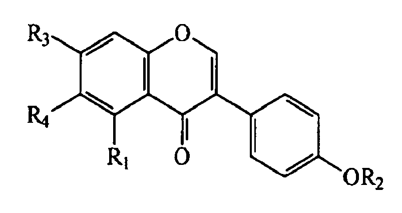

| **Isoflavonglucosides** | **R₁** | **R₂** | **R₃** | **R₄** |
|---|---|---|---|---|
| Daizidin | H | H | Glucose | H |
| Genistin | H | H | Glucose | OH |
| Ononin | H | CH₃ | Glucose | H |
| Sissostrin | H | CH₃ | Glucose | OH |

### Litchi sinensis

Extracts of pericarps from Litchi (*Litchi sinensis*) are well known for its high content of flavon derivatives like e.g. 2-phenyl-4H-1-benzopyrans, flavanen, flavan-3-ols (catechins, catechin oligomeren), flavan-3,4-diols (leucoanthocyaniden), flavons, flavonols and flavonons. The main component, however, represent condensed tannins, so-called procyanodols (OPC). These compounds comprise 2 to 8 monomers of the catechin or epicatechin-type, like e.g. procyanidins, proanthocynidins, procyanidoel, oligoprocyanidins, leucoanthocyanidins, leucodelphinins, leucocyanins and anthocyanogens. OPC, mainly the preferred proanthocyanidin A2 (OPC A2) behave like vitamin P, especially with respect to MMP inhibition.

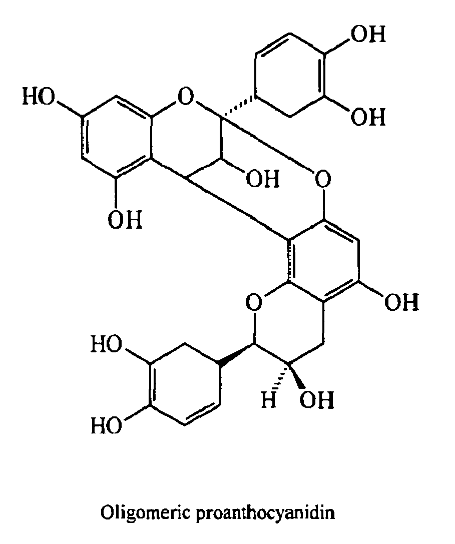

### Vitis vinifera

The main actives of grape vine (*Vitis vinifera*) are polyphenols of the OPC type.

### Brassica oleracea

The main active principles of cauliflower (*Brassica oleracea*) are amino acids, especially methionin and cystein, and glucosinolates like e.g. glucoraphanin.

### Punica granatum

The main active principles of grenadine (*Punica granatum*) are sugars, citric acid and delphinidin-1,2-glykoside or its aglykon

### Petroselinium crispum

Main constituent of the fatty oil of parsil (*Petroselinium crispum*) is petroselinic acid. The extracts, however, show high contents of apiol (1-allyl-2,5-dimethoxy-3,4-(methylendioxy)benzol), and in addition of apiin, myristicin, pinen und selinen.

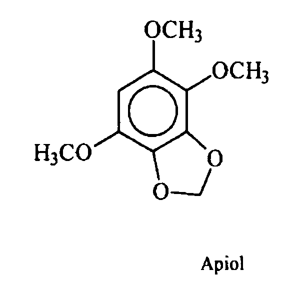

### Passiflora incamata

Extracts of passion flower (*Passiflora incarnata*) are rich in flavons of the apigenin and luteolin-type and their C-glycosides:

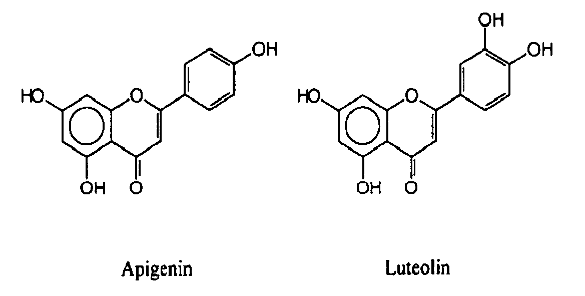

In addition they comprise 2"-B-D-glucosides, schaftosides and iso-schaftosides, isovitexin, isoorientin, vicenin-2, incenin-2, daponanin and trace elements like calcium, phosphor and iron.

### Medicago sativa

Extracts of Alfalfa (*Medicago sativa*) are rich in isoflavons like e.g. daidzein, genestein, formononetin, biochanin A und tricin :

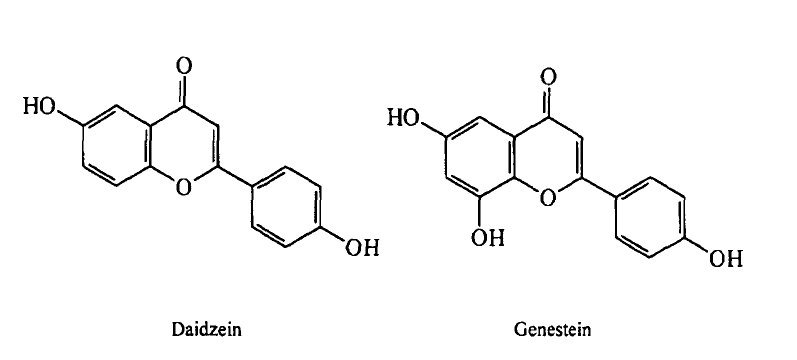

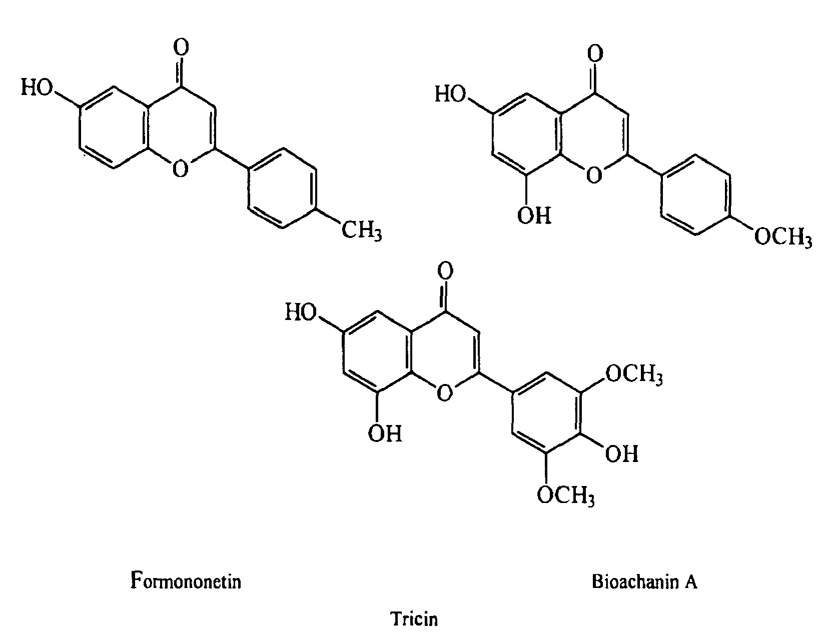

### Valeriana officinalis

The main constituents of extracts of *Valeriana officinalis* are valeric acid, valerianone and borneol esters.

### Castanea sativa

Main ingredients of horse chestnuts (*Castanea sativa*) are saponins and escin, which is a mixture of two glycosides, whose aglycons are derived from proteoescigenin, while the sugars represent either glucoronic acid of two molecules D-glucose. Said glycosides differ in the acyl groups in the C22-position.

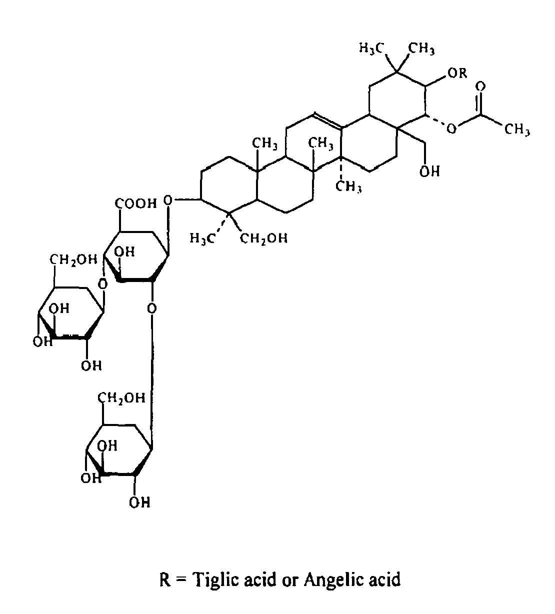

While α-escin represents an amorphous powder, which melts between 225 and 227 °C and is easily soluble in water, β-escin (which is also called flogencyl) forms flakes, which are practically water-insoluble, but can be dissolved in alcohol.

### Harpagophytum procumbens

The main active principles of devil's craw (*Harpagophytum procumbens*) are iridoidglucosides, harpagosides, harpagides and procumbides.

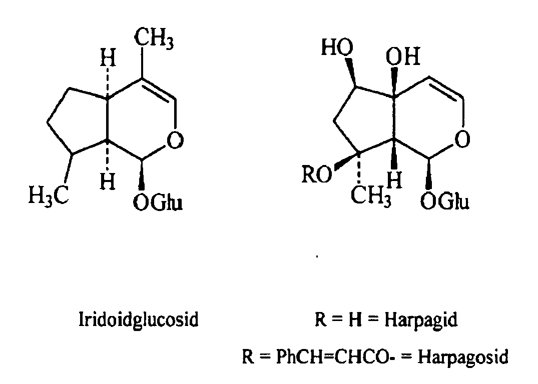

In addition one finds stachylose, free and glycosylated phytosterols (e.g. β-sitosterol), flavonoides (e.g. kaempferol, luteolin), phenolic acids und glycosidic phenylpropanoicacid esters (e.g. verbacosides, isoacteosides).

### Hoodia gordonii

*Hoodia* is a South African cactus plant that was traditionally used by the original population during hunting periods to suppress appetite. It has been found that this special property is linked to its content of active steroid glycosides. In 2001/2002 one of these actives, called Substance P57, was identified and isolated:

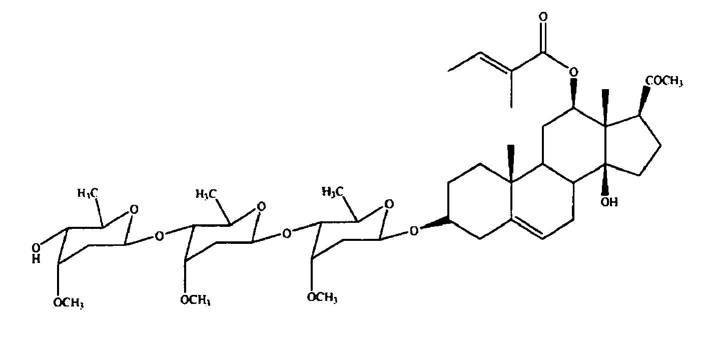

### Melissa officinalis

Main ingredients of lemon balm (Melissa officinalis) flavonoids and polyphenolic compounds as well as polyphenolic acids, mostly rosmarinic acid. Studies suggest that lemon balm may have a beneficial effect on mood resulting in significantly increased "calmness". Therefore it is a preferred combination with ingredients to support weight loss as the improved mood and calmness help to maintain the healthy diet.

### Panax ginseng C.A.

The main bioactive constituents of ginseng (Panax ginseng C.A) are considered to be triterpene saponins, generally referred to as ginsenosides. In vitro and in vivo behavioural studies suggest that ginseng and its component ginsenosides may improve indices of stress, fatigue, and learning.

### Cyclopia ssp L.

Honey bush (cyclopia ssp.) are characterized by several bioactive compounds including various antioxidant flavonoids (flavonols, flavones, flavanones, isoflavones), the xanthone mangiferin and the inositol derivative pinitol. It contains no caffeine and only very low amounts of tannins. Mangiferin may have numerous health-protecting effects, including immune boosting and antidiabetic, By improving blood sugar and lipid metabolism, it may be helpful in the treatment of weight control.

### Aspalathus linearis L.

The main ingredients of rooibos (*Aspalathus linearis L.*) are polyphenolic compounds, acidic polysaccharides and small amounts of essential oils. It is caffeine-free. The most important polyphenolic compounds are flavonoids, particularly orientin, isoorientin, rutin, aspalathin, luteolin and quercetin - believed to be the major active constituents. Rooibos is currently the only known natural source of aspalathin. It has a comparatively high content of minerals. Epidemiological studies indicate that a diet rich in polyphenols may lower the risk of atherosclerosis and other metabolic syndromerelated health conditions.

### Paullinia cupana

The main ingredient of guarana (Paullinia cupan.) is caffeine along with other minor xanthic bases, tannins and saponins.

### Opuntia ficus-indica

The main ingredients of prickly pear (Opuntia ficus-indica) are lipophilic fibres, which may reduce the gastrointestinal fat absorption. Adulterations with Opuntis are found in many today's Hoodia products on the US market.

### Caralluma fimbriata

Luteolin-4-O-neohesperidoside has been identified as "the major chemical constituent of the plant." In addition it is said to contain saponin glycosides. It is used to support weight loss and is said to be a appetite suppressum.

The extracts of Aloe on one side and the extracts of the plant extracts cited above can be used in ratios by weight of 10:90 to 99:1 and in particular 70:30 and 90:10.

### Physiologically active agents

In another preferred embodiment of the present invention the preparations, purifications and/or extracts of Aloe can be combined with physiologically active agents selected from the group consisting of chitosans, physiologically active fatty acids and their derivatives, sterols and sterol esters.

### Chitosans

Chitosans, for example, are biopolymers which belong to the group of hydrocolloids. Chemically, they are partly de-acetylated chitins differing in their molecular weights which contain the following - idealized - monomer unit:

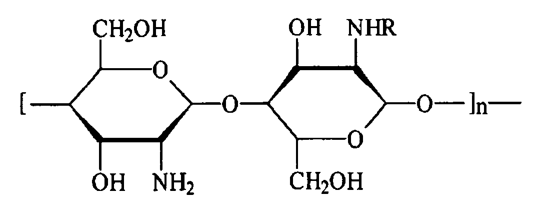

In contrast to most hydrocolloids, which are negatively charged at biological pH values, chitosans are cationic biopolymers under these conditions. The positively charged chitosans are capable of interacting with oppositely charged surfaces and are therefore used in cosmetic hair- care and body-care products and pharmaceutical preparations. Chitosans are produced from chitin, preferably from the shell residues of crustaceans which are available in large quantities as inexpensive raw materials. In a process described for the first time by Hackmann et al., the chitin is normally first de-proteinized by addition of bases, de-mineralized by addition of mineral acids and, finally, de-acetylated by addition of strong bases, the molecular weights being distributed over a broad spectrum. Preferred types are those which are disclosed in German patent applications DE 4442987 A1 and DE 19537001 A1 (Henkel) and which have an average molecular weight of 10,000 to 500,000 Dalton or 800,000 to 1,200,000 Dalton and/or a Brookfield viscosity (1 % by weight in glycolic acid) below 5,000 mPas, a degree of de-acetylation of 80 to 88 % and an ash content of less than 0.3% by weight. In the interests of better solubility in water, the chitosans are generally used in the form of their salts, preferably as glycolates.

### Physiologically active fatty acids, their salts and their esters

A common criteria for fatty acids with physiological activity, which represent component (b), is a fat chain having a sufficient number of carbon atoms providing a lipophilic behaviour that allows the molecule to pass through the gastrointestinal tract of the body and having a sufficient number of double bonds. Therefore, said fatty acids usually comprise 18 to 26 carbon atoms and 2 to 6 double bonds.

In a first embodiment of the present invention conjugated linoleic acid (CLA) or its alkaline or alkaline earth salts and esters, preferably, their calcium salts and their esters with lower aliphatic alcohols having 1 to 4 carbon atoms - or their glycerides, specially their triglycerides come into account. Conjugated linoleic acid (CLA) represents a commercially available product which usually is obtained by base-catalysed isomerisation of sunflower oil or their respective alkyl esters and subsequent isomerisation in the presence of enzymes. CLA is an acronym used for positional and geometric isomers deriving from the essential fatty acid linoleic acid (LA, cis-9,cis-12-octadecadienoic acid, 18:2n-6). From a physiological point of view the use of the *cis-9,trans*-11 isomer according to the present invention is of special importance having at least 30, preferably at least 50, and most preferably at least 80 % b.w. of said *cis*-9,*trans*-11 isomer - based on the total CLA content of the crude mixture. In addition, it has been found advantageous if the content of the *trans*-10,*cis*-12 isomer is at most 45, preferably at most 10 % b.w. and most preferably less than 1 % b.w., and the sum of 8,10-, 11,13- and *trans,trans-*isomers in total is less than 1 % b.w. - again based on the total CLA content. Such products can be found in the market, for example, under the trademark Tonalin^{®} CLA-80 (Cognis).

In a second embodiment also so-called omega-3 fatty acids can come into account, which typically comprise 18 to 26, preferably 20 to 22 carbon atoms and at least 4 and up to 6 double bonds. Also these molecules are very well known from the art and can be obtained by standard methods of organic chemistry, for example, via transesterification of fish oils, followed by urea precipitation of the alkyl esters thus obtained and a final extraction using non-polar solvents as described in the German patent DE 3926658 C2 (Norsk Hydro). Fatty acids thus obtained are rich in omega-3 (all-Z)-5,8,11,14,17-eicosapentanoic acid (EPA) C 20 : 5 and (all-Z)-4,7,10,13,16,19-docosahexanoic acid (DHA) C 22 : 6. Such products can be found in the market under the trademark Omacor® (Pronova).

In a third embodiment also linoleic acid, vaccinic acid (trans 11-octadecenoic acid), or cis-hexadecenoic acid (obtained, for example, from the plant *Thunbergia alata*) can be used.

In addition, said physiologically active fatty acid esters can not only be used in form of their lower alkyl esters or glycerides. An additional well preferred embodiment of the present invention relates to compositions comprising esters of said fatty acids with sterols. Like glycerides, sterol esters are easily resorbed and split by the human body. However, a significant advantage comes from the fact that the cleavage of the ester bond releases a second molecule with health promoting properties. To avoid unclarities, the phrases "sterol", "stanol", and "sterin" shall be used as synonyms defining steroids showing a single hydroxyl group linked to the C-3. In addition, sterols which consist of 27 to 30 carbon atoms, may show a double bond, preferably in 5/6 position. According to the present invention, esters of CLA or omega-3 fatty acids with β-sitosterol or its hydrogenation product β-sitostanol are preferred.

### Sterols and sterol esters

Sterols - also called sterins - represent steroids showing a single hydroxyl group linked to the C-3. In addition sterols, which consist of 27 to 30 carbon atoms, may show a double bond, preferably in 5/6 position. The hydrogenation of the double bond ("hardening") leads to sterols which are usually called stanols. The figure below shows the structure of the best known member of the sterol family, cholesterol, which belongs to the group of zoosterols.

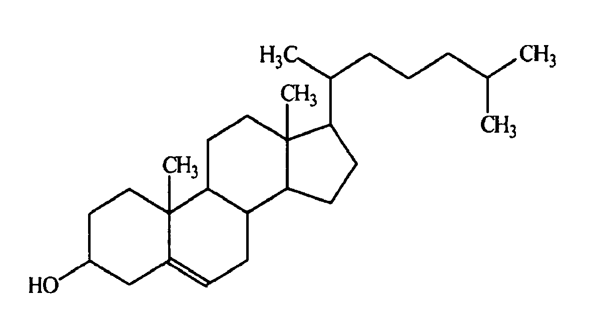

Due to their superior physiological activity, the plant sterols, so-called phytosterols, like ergosterol, stigmasterol, and especially sitosterol and its hydrogenation product sitastanol, are the preferred species. In addition instead of the sterols or stanols their esters with saturated or unsaturated fatty acids having 6 to 26 carbon atoms and up to 6 double bonds can be used. Typical examples are the esters of β-sitosterol or β-sitostanol with capric acid, caprylic acid, 2-ethylhexanoic acid, caprinic acid, lauric acid, isotridecylic acid, myristic acid, palmitic acid, palmoleic acid, stearic acid, isostearic acid, oleic acid, elaidinic acid, petroselinic acid, linolic acid, linoleic acid, elaeostearic acid, arachidonic acid, gadoleinic acid, behenic acid and erucic acid.

The preparations, purifications or extracts of Aloe on one side and the actives cited above can be used in ratios by weight of 10:90 to 99:1 and in particular 70:30 and 90:10.

### Industrial application

In a special embodiment of the present invention said extracts of Aloe are administered to the humans in a macro- or micro-encapsulated form. "Microcapsules" are understood to be spherical aggregates with a diameter of about 0.1 to about 5 mm which contain at least one solid or liquid core surrounded by at least one continuous membrane. More precisely, they are finely dispersed liquid or solid phases coated with film-forming polymers, in the production of which the polymers are deposited onto the material to be encapsulated after emulsification and coacervation or interfacial polymerization. In another process, liquid active principles are absorbed in a matrix ("microsponge") and, as microparticles, may be additionally coated with film-forming polymers. The microscopically small capsules, also known as nanocapsules, can be dried in the same way as powders. Besides single-core microcapsules, there are also multiple-core aggregates, also known as microspheres, which contain two or more cores distributed in the continuous membrane material. In addition, single-core or multiple-core microcapsules may be surrounded by an additional second, third etc. membrane. The membrane may consist of natural, semisynthetic or synthetic materials. Natural membrane materials are, for example, gum arabic, agar agar, agarose, maltodextrins, alginic acid and salts thereof, for example sodium or calcium alginate, fats and fatty acids, cetyl alcohol, collagen, chitosan, lecithins, gelatin, albumin, shellac, polysaccharides, such as starch or dextran, polypeptides, protein hydrolyzates, sucrose and waxes. Semisynthetic membrane materials are inter alia chemically modified celluloses, more particularly cellulose esters and ethers, for example cellulose acetate, ethyl cellulose, hydroxypropyl cellulose, hydroxypropyl methyl cellulose and carboxymethyl cellulose, and starch derivatives, more particularly starch ethers and esters. Synthetic membrane materials are, for example, polymers, such as polyacrylates, polyamides, polyvinyl alcohol or polyvinyl pyrrolidone. Examples of known microcapsules are the following commercial products (the membrane material is shown in brackets) *Hallcrest Microcapsules* (gelatin, gum arabic), *Coletica Thalaspheres* (maritime collagen), *Lipotec Millicapseln* (alginic acid, agar agar), *Induchem Unispheres* (lactose, microcrystalline cellulose, hydroxypropylmethyl cellulose), *Unicerin C30* (lactose, microcrystalline cellulose, hydroxypropylmethyl cellulose), *Kobo Glycospheres* (modified starch, fatty acid esters, phospholipids), *Softspheres* (modified agar agar), *Kuhs Probiol Nanospheres* (phospholipids) and *Primaspheres* or *Primasponges* (chitosan, anionic polymers).

It is of course also possible to incorporate the extracts directly into a food composition, for example a beverage, a yoghurt, a milk, a milk shake or a candy snack.

### Examples

### Influence of extracts on feeding behaviour and responses of an insect, Spodoptera littoralis, to glucose

The feeding behaviour of insects is modulated by the levels of nutrients in their diet as well as "secondary metabolites" from plants. It is known that an insect that has been given a high carbohydrate diet becomes less responsive to sucrose or glucose, whereas the response to protein is not influenced to the same level. The insect model has been applied for studying whether plant preparations and/or extracts would alter the feeding behaviour of the insect and especially alter their response to glucose. Due to the fact that there are many similarities in how insects and mammals perceive compounds, the results how plant derived preparations and extracts decrease the responsiveness to a phagostimulant and thus decrease food intake are indicators whether or said plant extracts could also be useful to support satiety in humans or reduce appetite in order to reduce calories intake, to control and to reduce weight, especially body fat.

All experiments were done on the final stadium larvae of *Spodoptera littoralis.* Prior to the experiment the insects were deprived for food for 2 hours. This has been calculated as a period when most insects would start to feed. Thus the food from the previous meal has started to pass into and through the gut.

The initial experiment investigated whether the insects could "taste" the plant preparations or extracts and whether they stimulated or deterred feeding. A binary choice experiment with glass-fibre discs treated with a phagostimulant (sucrose or glucose at 0.05M) and a test plant preparation or extract were used to test whether the extracts modulate insect feeding. The amount eaten of a control disc (C: treated with a phagostimulant) and a treatment disc (T: treated with a phagostimulant and a test extract; preparation or extracts tested at concentrations 100 ppm, 1000 ppm and 10,000 ppm) after 18 hours was used to calculate a Feeding Index (FI) ((C-T)/(C+T)) %. A negative value indicates a phagostimulant and a positive value indicates an anti-feedant. Experiments are usually repeated with between 10-20 insects and the data are analysed using the Wilcoxon matched pairs test. A series of controls were used at the start of the experiment to illustrate the difference in the response of the larvae to sucrose and glucose both tested at 0.05M. Results have been presented as means.

### Examples I - V

### Responses to sugars

The data presented in Table 1 shows the response of insects to a series of different combinations of sugar-treated and untreated discs. When insects were exposed to a choice between two discs treated with the same stimulant they did not discriminate, resulting in a low FI. In contrast, when the insects were exposed to control discs treated with a sugar and the treatment disc was just treated with water they ate more of the sugar-treated disc than the blank disc. The data also shows that the response to sucrose was greater than that to glucose which shows that sucrose is a more potent phagostimulant to *S*. *littoralis* than glucose. Because sucrose is more potent than glucose the next series of experiments with the test extracts were tested in combination with sucrose and then repeated with glucose.

**Table 1**

| **Effect of sugars on feeding response of *Spodoptera littoralis*** | | | |
|---|---|---|---|
| **Example** | **Test: Control versus Treatment** | | **Feeding Index** |
| I | Sucrose | Sucrose | 10.7 |
| II | Glucose | Glucose | 8.7 |
| III | Sucrose | Glucose | 23.3 |
| IV | Sucrose | untreated | 46.3* |
| V | Glucose | untreated | 29.5* |

| | | | |
|---|---|---|---|
| Sucrose and glucose tested at 0.05M; Feeding Index = ((C-T)/(C+T)) %, n=15; * = P<0.05 Wilcoxon matched pairs test | | | |

### Examplesl-2, Comparison Examples C1-C8

### Responses to extracts in combination with either sucrose or glucose

**(i) Sucrose.** The responses of *S*. *littoralis* to the extracts varied (Table 2a). The Aloe and Olive leaf extracts were the only two extracts to elicit a significant phagostimulant response across all three concentrations. This indicates that the insects fed on the disc treated with either Aloe or olive leaf in preference to the disc treated with sucrose. The 0.05M concentration was selected for the feeding experiments as this concentration was optimal. Ginseng was an antifeedant at all concentrations tested, this was the only extract to show antifeedant activity at the three concentrations.
**(ii) Glucose.** The responses of *S*. *littoralis* to the extracts and other test material when tested in combination with glucose were similar to those recorded with sucrose, although the level of activity was sometimes greater (Table 2b). For example, the FI response of *S*. *littoralis* to 100 ppm Aloe when combined with sucrose was FI =-25, whereas when combined with glucose the response was FI = - 42. When combined with glucose both aspartame and saccharin stimulated feeding at lower concentrations, whereas with sucrose the response had not been significant. As with sucrose, ginseng was an antifeedant at all three concentrations.

**Table 2a**

| **Effect of different test extracts on the feeding behaviour of *S. littoralis* when tested in combination with sucrose** | | | | | |
|---|---|---|---|---|---|
| **Ex.** | **Control** | **Extracts** | **Feeding Index** | | |
| | | | **10,000 ppm** | **1,000 ppm** | **100 ppm** |
| 1 | Sucrose | Aloe Vera | -48* | -35* | -25* |
| C1 | | Ginseng | 35* | 29* | 28* |
| C2 | | Olive leaves | -25* | -24* | -24* |
| C3 | | Aspartame | 15 | 14 | -15 |
| C4 | | Saccharin | 5 | -5 | -16 |

| | | | | | |
|---|---|---|---|---|---|
| Sucrose tested at 0.05M; Feeding Index = ((C-T)/(C+T)) %, n=15; * = P<0.05 Wilcoxon matched pairs test | | | | | |

**Table 2b**

| **Effect of different test extracts on the feeding behaviour of *S. littoralis* when tested in combination with glucose** | | | | | |
|---|---|---|---|---|---|
| **Ex.** | **Control** | **Extracts** | **Feeding Index** | | |
| | | | **10,000 ppm** | **1,000 ppm** | **100 ppm** |
| 2 | Glucose | Aloe Vera | -49* | -49* | -42* |
| C5 | | Ginseng | 46* | 35* | 26* |
| C6 | | Olive leaves | -25* | -28* | -49* |
| C7 | | Aspartame | -24 | -31* | -25* |
| C8 | | Saccharin | -18 | -25* | -24* |

| | | | | | |
|---|---|---|---|---|---|
| Glucose tested at 0.05M; Feeding Index = ((C-T)/(C+T)) %, n=15; * = P<0.05 Wilcoxon matched pairs test | | | | | |

### Example3, Comparison Examples C9-C12

### Response of cannulated insects to glucose

**Material and methods.** Final stadium larvae were removed from food and after 2 hours they were cannulated via the oral cavity with 1.5 ml of a test solution, water was used as a control. The extracts were tested at 10,000 ppm, 1,000 ppm and 100 ppm. The insects were then placed in a Petri dish with a glucose treated glass-fibre disc. The time taken for the insects to start a meal (latency), the duration of a meal (meal duration) and the time taken to take the second meal were recorded. This provides information about whether the extracts once consumed would delay feeding, whether the extracts would alter the duration of a meal once it had started and whether the time to the second meal would be altered. An extract that modulates feeding behaviour might act in different ways: delay the start of a meal (decrease in appetite to feed), shorten the meal (satisfied after eating less), influence further feeding (influence of extract is not short term (30-90 minutes). A meal starts when the insects have eaten for more than 90 seconds and terminates when the insects stop feeding for a 90 second period. Data are presented in minutes and are the mean values of 10 insects and have been rounded to the nearest minute.
**(i) Latency.** The time to the start of a meal was extended, when compared with the water control, after cannulation with Aloe and Ginseng. Aloe acted as stimulants in the glass-fibre disc bioassays, whereas Ginseng was a potent anti-feedant. Thus the Ginseng could be having a negative activity on time to feed, whereas the response to Aloe could be because of a "positive" response. The end effect is the same that both groups cause an increased in the latency to feed. None of the extracts resulted in a decrease in the time to feed i.e. increased appetite.
**(ii) Meal Duration.** None of the extracts influenced the duration of the first recorded meal. Thus although insects cannulated with Aloe delayed feeding once they started to feed the duration of the meal was similar to that taken by the water or glucose treated insects.
**(iii) Time to second meal.** All the concentrations of Aloe increased the time taken before the insects had their second meal. In contrast, the greatest concentration of Ginseng tested resulted in a decrease in the time to the second meal. This could indicate that the "adverse" response that resulted in the initial delay to feeding is no longer being experienced by the insect and it is compensating for a decrease in food intake by decreasing the time between meals rather than increasing meal duration.

**Table 3**

| **Influence of compounds on the behavioural response of larvae of *Spodoplera littoralis*** | | | | | | | | | | |
|---|---|---|---|---|---|---|---|---|---|---|
| **Ex.** | **Extract Concentration** | **Latency [min]** | | | **Meal duration [min]** | | | **Time to 2^{nd} meal [min]** | | |
| | | **10k** | **1k** | **0.1k** | **10k** | **1k** | **0.1k** | **10k** | **1k** | **0.1k** |
| VI | Glucose | 25 | 10 | 10 | 9 | 10 | 12 | 25 | 20 | 15 |
| 3 | Aloe Vera | 78* | 75* | 30* | 10 | 7 | 8 | 40* | 45* | 38* |
| C9 | Ginseng | 90* | 60* | 30* | 15 | 16 | 14 | 10* | 11 | 15 |
| C10 | Olive leaves | 15 | 16 | 17 | 14 | 12 | 17 | 14 | 15 | 14 |
| C11 | Aspartame | 20 | 24 | 21 | 12 | 12 | 17 | 17 | 15 | 15 |
| C12 | Saccharin | 25 | 24 | 24 | 14 | 14 | 16 | 18 | 18 | 11 |

| | | | | | | | | | | |
|---|---|---|---|---|---|---|---|---|---|---|
| Solvent: water; * = P<0.05 Wilcoxon matched pairs test | | | | | | | | | | |

### Example 4, Comparison Examples C13-C16

### Electrophysiology: neural response to glucose

Insects use sensilla on their mouthparts to taste their food. These sensilla contain neurones that respond to compounds in their food. An electrophysiological bioassay has been developed to record the neural impulses from the four neurones in each sensilla. The sensilla are stimulated for 1 sec with a test solution and the number of impulses recorded. Previous experiments have shown that the responsiveness of the insects can be influenced by the diet an insect has been reared on and previous exposure to a compound. The physiological condition of the insect can also influence the responsiveness of these neurones. In these experiments we can classify the neurones down to specific neurones but this requires some further experiments not undertaken in this study. In this experiment we tested the medial sensilla and recorded the mean total response to a 1 sec stimulation. Each larva was tested with KCl (C= 0.05M) as a control, sucrose (S= 0.05M), glucose (G = 0.05M) and amino acid mix (P =0.05M). A set of experiments were undertaken on a control non-cannulated group of insects. All treatment insects were cannulated with 1.5ml of a test extract/product at 1000 ppm and then tested after 30, 60 and 90 minutes. Prior to the experiment the insects had been standardised as in the behavioural experiments. There were 5-10 replicates per extract.

**Table 4**

| **Neural responses (impulses per second) to stimulation of the medial sensilla of *Spodoptera littoralis* larvae** | | | | | | | | | | | | | |
|---|---|---|---|---|---|---|---|---|---|---|---|---|---|
| **Ex.** | **Extract cannulation** | **Impulses per second from the medial styloconic sensilla** | | | | | | | | | | | |
| | | **30 min** | | | | **1 h** | | | | **3 h** | | | |
| | | **S** | **G** | **P** | **C** | **S** | **G** | **P** | **C** | **S** | **G** | **P** | **C** |
| VII | Control | 57 | 35 | 32 | 10 | 65 | 45 | 28 | 10 | 74 | 54 | 38 | 12 |
| VIII | Glucose | 68 | 12* | 25 | 8 | 85 | 18* | 24 | 10 | 75 | 29* | 45 | 10 |
| IX | Protein | 45 | 27 | 8* | 6 | 48 | 31 | 15* | 9 | 87 | 45 | 41 | 8 |
| 4 | Aloe Vera | 14* | 15* | 5* | 8 | 25* | 24* | 21 | 8 | 24* | 21* | 35 | 5 |
| C13 | Ginseng | 45 | 48 | 24 | 9 | 56 | 28 | 41 | 12 | 84 | 65 | 24* | 8 |
| C14 | Olive leaves | 54 | 48 | 24 | 9 | 74 | 57 | 41 | 15 | 84 | 45 | 28 | 11 |
| C15 | Aspartame | 57 | 45 | 24 | 10 | 48 | 25 | 24 | 12 | 48 | 24* | 28 | 9 |
| C 16 | Saccharin | 65 | 45 | 29 | 9 | 48 | 28 | 47 | 15 | 90 | 42 | 32 | 10 |

| | | | | | | | | | | | | | |
|---|---|---|---|---|---|---|---|---|---|---|---|---|---|
| S = sucrose (0.05M), G = Glucose (0.05M), P = amino acid mixture(47,40,30,40,58,45,20,50 ratio of L amino acids leucine: glutamine, serine, methionine, phenyalanine, lysine,valine,alanine) C= KCI (0.05M) N= 5-10; * = P<0.05 Wilcoxon matched pairs test | | | | | | | | | | | | | |

As one can see from Table 4, the neural responses to sucrose and glucose in insects cannulated with Aloe were lower than the responses of the untreated insects. A decrease in neural firing would suggest that the insects are less responsive to the compounds and this correlates with the behavioural data. Those insects cannulated with glucose had a lower response to glucose over the 90 minute experimental period.

### Conclusion

These experiments have shown that the insect model does show that exposure to the extracts of Aloe influences the behavioural and neural responses of *S. littoralis* to glucose as well as other compounds. The model also shows that some other extracts also influence behaviour but this could be because the extracts are antifeedant (i.e "do not taste nice").

## Claims

1. Use of preparations, purifications and/or extracts of Aloe as dietary supplements or functional food.

2. Use according to Claim 1, **characterised in that** the dietary supplement or functional food is an oral composition for controlling or reducing weight or to optimize the body composition

3. Use according to Claim 1, **characterised in that** the dietary supplement or functional food is an oral composition for supporting satiety of mammals.

4. Use according to Claim 1, **characterised in that** the dietary supplement or functional food is an oral composition for decreasing appetite.

5. Use according to any of the Claims 1 to 4, **characterised in that** said preparations, purifications or extracts of Aloe are present in the final composition in amounts of from 0.1 to 99 % b.w.

6. Use according to any of the Claims 1 to 5, **characterised in that** said preparations and extracts are incorporated into the final composition as gels or powders.

7. Use according to any of the Claims 1 to 6, **characterised in that** said preparations, purifications and/or extracts of Aloe are combined with extracts of plants selected from the group consisting of *Ginkgo biloba, Oleacea europensis, Panex ginseng, Trifolium pratense, Litchi sinensis, Vitis vinifera, Brassica oleracea, Punica granatum, Petroselinium crispum, Passiflora incarnata, Medicago saliva, Valeriana officinalis, Castanea sativa, Hapagophytum procumbens, Melissa officinalis, Camelia sinensis, Paulina cupana, Opuntia ficus India, Caralluma fimbriata, Aspalathus linearis,* and *Cyclopia ssp*

8. Use according to any of the Claims 1 to 7, **characterised in that** said preparations, purifications and/or extracts of Aloe are combined with physiologically active agents selected from the group consisting of chitosan, physiologically active fatty acids and their derivatives, sterols and sterol esters.

9. Use according to any of the Claims 1 to 8, **characterised in that** said preparations and extracts are administered in a macro- or micro-encapsulated form.
